(19) **Europäisches Patentamt European Patent Office Office européen des brevets**

(11) **EP 3 814 196 B1**

(12) **FASCICULE DE BREVET EUROPEEN**

(45) Date de publication et mention de la délivrance du brevet:
**15.05.2024 Bulletin 2024/20**

(21) Numéro de dépôt: **19730388.6**

(22) Date de dépôt: **19.06.2019**

(51) Classification Internationale des Brevets (IPC):
**B62D 1/04** *(2006.01)*   **B62D 1/06** *(2006.01)*
**G01R 35/00** *(2006.01)*   **G01R 27/02** *(2006.01)*
**B60W 40/08** *(2012.01)*   **G01D 18/00** *(2006.01)*
**G01V 13/00** *(2006.01)*   **A61B 5/00** *(2006.01)*
**G01V 3/08** *(2006.01)*   **G01R 31/00** *(2006.01)*
**G01R 27/26** *(2006.01)*   **B60R 21/015** *(2006.01)*
**G01D 5/24** *(2006.01)*

(52) Classification Coopérative des Brevets (CPC):
**B62D 1/046; A61B 5/6893; B62D 1/065;
G01R 27/02; G01R 27/2605; G01R 31/006;
G01R 35/00; G01V 3/08; G01V 13/00;
A61B 2562/0257; B60R 21/0152**

(86) Numéro de dépôt international:
**PCT/EP2019/066177**

(87) Numéro de publication internationale:
**WO 2020/002079 (02.01.2020 Gazette 2020/01)**

(54) **DISPOSITIF CAPACITIF DE DÉTECTION DE LA PRÉSENCE D'UNE PERSONNE À PROXIMITÉ OU AU CONTACT D'UN COMPOSANT D'UN VÉHICULE AUTOMOBILE**

KAPAZITIVE VORRICHTUNG ZUR DETEKTION DER ANWESENHEIT EINER PERSON IN DER NÄHE ODER IM KONTAKT MIT EINEM AUTOMOBIL-ELEMENT

CAPACITIVE DEVICE FOR DETECTING THE PRESENCE OF A PERSON NEAR OR IN CONTACT WITH AN ELEMENT OF AN AUTOMOBILE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.06.2018 FR 1855743**

(43) Date de publication de la demande:
**05.05.2021 Bulletin 2021/18**

(73) Titulaire: **Autoliv Development AB
447 83 Vårgårda (SE)**

(72) Inventeur: **SABOURIN, Pierre
78920 Ecquevilly (FR)**

(74) Mandataire: **Poindron, Cyrille
Novagraaf International SA
Chemin de l'Echo 3
1213 Onex (CH)**

(56) Documents cités:
**EP-A2- 0 802 420    WO-A1-2016/055667**

EP 3 814 196 B1

**Description**

La présente invention concerne de manière générale un dispositif capacitif de détection pour véhicule automobile destiné à détecter la présence d'une personne à proximité ou au contact d'un composant du véhicule. Le dispositif peut notamment servir à détecter la présence des mains d'un conducteur sur le volant du véhicule ou la présence d'un occupant sur l'un des sièges du véhicule.

Il est connu dans l'art antérieur de tels dispositifs capacitifs, notamment dans le document US 2015/0367751 qui décrit un dispositif capacitif pour la détection de présence d'une personne sur un siège de véhicule. Le dispositif capacitif décrit dans US 2015/0367751 comporte une électrode « antenne » ou « électrode de détection » disposée dans le siège, une source de tension alternative et un circuit de mesure de courant. La source de tension alternative et le circuit de mesure de courant sont connectés à l'électrode antenne. Une partie châssis du siège, connectée à la masse électrique du véhicule, fait fonction de seconde électrode couplée à l'électrode antenne pour former un capteur capacitif. La mesure d'une impédance complexe entre l'électrode antenne et la masse électrique du véhicule permet de détecter la présence d'une personne sur le siège. Le dispositif capacitif comprend en outre une impédance normative connue couplée à un élément de commutation. Lors d'un fonctionnement en mode mesure, le circuit de mesure de courant mesure le courant alternatif circulant entre l'électrode antenne et la masse électrique et en entrée d'un amplificateur AOP à transimpédance ayant une fonction de convertisseur courant-tension. L'impédance complexe recherchée entre l'électrode antenne et la masse électrique peut être calculée à partir d'une tension de sortie de l'amplificateur à transimpédance, du signal de sortie complexe de la source de tension et d'un facteur de proportionnalité $\alpha$. Afin de déterminer ou d'éliminer ce facteur de proportionnalité $\alpha$, la mesure réalisée en mode mesure est répétée lors d'un fonctionnement du dispositif capacitif en mode calibration. En mode calibration, l'impédance normative est commutée électriquement en parallèle de l'impédance à déterminer entre l'électrode antenne et la masse électrique. Cette impédance recherchée entre l'électrode antenne et la masse électrique est ensuite calculée à partir des tensions de sortie complexes de l'amplificateur à transimpédance mesurées en mode mesure et en mode calibration et de l'impédance normative.

Le document US 2017/355337 décrit un circuit de mesure d'impédance complexe pour un capteur capacitif à deux électrodes. Afin de calculer les valeurs absolues des parties réelle et imaginaire de l'impédance complexe du capteur capacitif, une opération de normalisation en amplitude et de correction de phase est réalisée en multipliant la partie réelle et la partie imaginaire de l'impédance complexe mesurée par un vecteur de calibration déterminé a priori. Le document WO2016055667A1 divulgue un dispositif de détection capacitif. Le document EP0802420A2 divulgue un dispositif de mesure d'impédance.

Toutefois, l'art antérieur ne permet pas de réaliser une mesure d'impédance, ou d'admittance, complexe avec précision. La présente invention vient améliorer la situation. A cet effet, l'invention concerne un dispositif de détection capacitif pour détecter la présence d'une personne à proximité ou en contact avec un composant d'un véhicule automobile selon la revendication indépendante 1.

L'invention concerne également un dispositif de détection capacitif pour détecter la présence d'une personne à proximité ou au contact avec un composant d'un véhicule automobile selon la revendication indépendante 3.

L'électrode du dispositif de détection capacitif appartient à un capteur capacitif équipant le composant du véhicule à proximité ou au contact duquel la détection de présence d'une personne est destinée à être réalisée. Le capteur capacitif comprend deux électrodes séparées par un matériau diélectrique.

Avantageusement, le dispositif de détection capacitif comporte l'une des deux électrodes d'un capteur capacitif et le composant du véhicule comporte l'autre électrode du capteur capacitif.

Avantageusement encore, l'une au moins des électrodes du capteur capacitif peut être réalisée par un élément structurel du composant du véhicule. Par exemple, une telle électrode peut être réalisée par un élément d'armature du composant du véhicule.

Ledit dispositif de détection capacitif est caractérisé en ce qu'il comprend un dispositif de commutation agencé pour:

- connecter la source de tension à ladite électrode, en mode mesure,
- connecter la source de tension à la résistance de calibration et déconnecter ladite électrode de la source de tension, en mode calibration,

et le dispositif de mesure est agencé pour :

- mesurer une première valeur complexe de la résistance de calibration, lors d'un fonctionnement en mode calibration ;
- mesurer une deuxième valeur complexe entre ladite électrode et le point de référence de circuit électrique, lors d'un fonctionnement en mode mesure, et
- corriger la deuxième valeur complexe mesurée en fonction de la première valeur complexe mesurée.

Ainsi, en mode calibration, la source de tension est connectée de façon sélective à la résistance de calibration et déconnectée de l'électrode. De façon facultative, lorsque la source de tension est connectée à l'électrode, elle peut être déconnectée de la résistance de calibration. La résistance de calibration, dont la valeur résistive est déterminée (calibrée) et connue, est utilisée pour faire une mesure d'impédance ou d'admittance complexe lors d'un fonctionnement en mode calibration (autrement dit en commutant la source de tension uniquement sur ladite résistance de calibration). La valeur complexe mesurée (d'impédance ou d'admittance) de la résistance de calibration permet de rectifier la mesure de la valeur complexe recherchée d'impédance ou d'admittance (entre l'électrode de détection et la masse) mesurée par le dispositif de mesure en mode mesure (c'est-à-dire en commutant la source de tension sur l'électrode). L'invention permet, par ce système de calibration, de compenser efficacement toute dérive de la mesure due des variations climatiques (température et humidité notamment) ou prendre en compte les variabilités de fonctionnement des composants électroniques utilisés pour la fabrication du dispositif. Selon l'invention, le dispositif capacitif de détection comprend deux résistances de calibration.

L'utilisation de deux résistances de calibration permet de réaliser deux mesures de valeurs complexes d'impédance ou d'admittance de deux dipôles électriques ayant des valeurs résistives différentes. Deux points de mesure différents sont ainsi obtenus dans un plan complexe (d'impédance ou d'admittance).

Dans une première forme de réalisation selon la revendication 1, les deux résistances de calibration ont des valeurs résistives différentes. Dans ce cas, le dispositif mesure séparément la valeur complexe (d'impédance ou d'admittance) de chacune des deux résistances de calibration. Avantageusement, les deux résistances de calibration sont montées en parallèle et le dispositif de commutation est agencé pour connecter la source de tension de façon sélective à l'une ou l'autre des deux résistances de calibration.

Par exemple, une première des résistances de calibration a une valeur résistive comprise entre 1 kΩ et 20 kΩ, préférentiellement entre 5 kΩ et 15 kΩ, et une deuxième des résistances de calibration a une valeur résistive comprise entre 10 kΩ et 200 kΩ, préférentiellement entre 50 kΩ et 150 kΩ. Ces deux plages de valeurs sont définies pour être proches respectivement de la valeur d'impédance complexe ajoutée par deux mains sur le volant et de la valeur d'impédance complexe ajoutée par un doigt sur le volant.

Dans une deuxième forme de réalisation selon la revendication 3, les deux résistances sont identiques. Dans ce cas, le dispositif mesure par exemple la valeur complexe d'impédance ou d'admittance de l'une seule des deux résistances de calibration et la valeur complexe d'impédance ou d'admittance des deux résistances de calibration montées en série.

En toute hypothèse, l'obtention de deux points de mesure de calibration distincts correspondant à deux dipôles électriques de valeurs résistives différentes permet de facilement corriger la phase de la mesure du fait que les deux valeurs complexes de calibration mesurées doivent nécessairement être alignées avec le zéro du plan complexe d'impédance ou d'admittance.

Avantageusement, le dispositif de mesure est agencé pour appliquer une correction d'offset à la deuxième valeur complexe mesurée, ladite correction d'offset correspondant à une translation selon un vecteur $\overrightarrow{S_{OC}O}$ dans un plan complexe, le point Soc étant un point du plan complexe correspondant à une valeur complexe d'admittance ou d'impédance en circuit ouvert du dispositif capacitif de détection et le point O correspondant à l'origine du plan complexe. La correction d'offset ou par décalage permet de corriger certains effets perturbateurs des différents étages de l'électronique d'émission ou de réception ainsi que des câbles de connexion du dispositif capacitif de détection sur la mesure d'impédance complexe. La présente invention permet ainsi de garantir le zéro des mesures d'impédance complexe.

Avantageusement encore, le dispositif de mesure est agencé pour calculer ladite valeur complexe d'impédance ou d'admittance en circuit ouvert du dispositif capacitif de détection à partir de deux valeurs complexes mesurées à l'aide des deux résistances de calibration. Le dispositif de mesure d'impédance complexe peut être agencé pour mesurer une valeur complexe (d'impédance ou d'admittance) en circuit ouvert et comparer ladite valeur complexe en circuit ouvert mesurée et la valeur complexe (d'impédance ou d'admittance) en circuit ouvert calculée afin de vérifier le bon fonctionnement du dispositif capacitif de détection.

Avantageusement encore, le dispositif de mesure est agencé pour appliquer une correction de phase à la deuxième valeur complexe mesurée, ladite correction de phase correspondant à une rotation selon un angle α entre une droite définie par deux points dans un plan complexe, obtenus à partir de deux valeurs complexes d'impédance ou d'admittance mesurées à l'aide des deux résistances de calibration, et un axe des résistances ou des conductances du plan complexe. Par « résistances de calibration », on entend ici désigner deux résistances de valeurs résistives connues ou éventuellement une résistance de valeur résistive connue et une résistance de valeur infinie équivalente à un circuit ouvert. Les résistances sont choisies pour avoir une valeur de résistance plus précise (de l'ordre de 0.1%) que le reste des autres composants. Elles sont aussi choisies pour avoir une très faible dérive en température (de l'ordre de 15ppm) et/ou une meilleure tenue aux contraintes mécaniques ou thermiques. Elles peuvent par exemple être de technologie « thin film » qui permet d'obtenir de meilleures performances que la technologie « thick film » habituellement utilisée. En effet, une résistance de technologie « thick film » évolue de manière plus importante lors d'un passage dans un four.

Avantageusement, le dispositif de mesure est agencé pour appliquer l'une au moins des deux corrections comportant une correction d'offset et une correction de phase, telles que précédemment définies. En d'autres termes, le dispositif de mesure peut être agencé pour appliquer soit une correction d'offset, soit une correction de phase, soit une correction d'offset et une correction de phase.

Avantageusement encore, le dispositif capacitif de détection comprend une paire de capacités de protection en série, ladite paire de capacités étant montée en parallèle de la ou des résistances de calibration et l'une des deux capacités est reliée à ses bornes à la source de tension par l'intermédiaire de deux commutateurs respectifs et l'autre capacité est reliée à une masse électrique du véhicule. Les capacités de protection permettent de protéger les résistances de calibration, qui sont de préférence des résistances de précision plus sensibles que les résistances classiques, contre les effets d'ESD (« electrostatic discharge ») ou de décharge électrostatique. Avantageusement, les valeurs capacitives des capacités de protection sont choisies dans des gammes proches des valeurs des capacités de construction du dispositif capacitif de détection. Ainsi les courants mesurés en mode calibration sont similaires à ceux mesurés en mode mesure. Cela garantit une précision de mesure optimale.

Le dispositif capacitif de détection peut être agencé pour répéter la mesure de la deuxième valeur complexe entre l'électrode et un point de référence de circuit électrique de façon cyclique et, entre deux mesures successives de ladite deuxième valeur complexe, exécuter une seule mesure de calibration.

Au préalable, lors d'une phase de calibration initiale, le dispositif peut être agencé pour faire deux mesures de calibration d'une première valeur complexe d'impédance ou d'admittance de deux dipôles électriques différents réalisés à partir des deux résistances de calibration.

L'invention permet ainsi de garantir une calibration continue dans le temps de la mesure d'impédance complexe et de compenser continuellement tout éventuel effet perturbateur (par exemple la température), sans dégrader le rythme des mesures de l'impédance complexe entre l'électrode et le point de référence de circuit électrique.

Avantageusement, le dispositif de mesure est agencé pour calculer une valeur de capacité entre l'électrode et un point de référence de circuit électrique du véhicule, à partir de ladite deuxième valeur complexe (d'impédance ou d'admittance) mesurée puis corrigée, par application d'un gain de mise à l'échelle G à la partie imaginaire de ladite deuxième valeur complexe mesurée puis corrigée, le gain G étant donné par la relation

$$G = \frac{|R_{C1} - R_{C2}|}{R_{C1} \times R_{C2} \times \left\| \overline{C_{R\_C1} C_{R\_C2}} \right\|}$$

où

- $R_{C1}$ représente une première résistance de calibration ;
- $R_{C2}$ représente une deuxième résistance de calibration ;
- $C_{R\_C1}$ représente un point, dans un plan complexe d'admittance, correspondant à la mesure d'une valeur complexe (d'impédance ou d'admittance) de la première résistance de calibration $R_{C1}$ ;
- $C_{R\_C2}$ représente un point, dans ledit plan complexe d'admittance, correspondant à la mesure d'une valeur complexe (d'impédance ou d'admittance) de la deuxième résistance de calibration $R_{C2}$.

Le dispositif de mesure comprend par exemple un circuit de mesure de courant.

L'invention concerne aussi un volant de véhicule équipé du dispositif capacitif de détection précédemment défini.

L'invention concerne également un siège de véhicule automobile équipé du dispositif capacitif de détection précédemment défini.

L'invention concerne encore un véhicule automobile comportant un volant et/ou un siège tels que définis ci-dessus.

D'autres caractéristiques et avantages de la présente invention apparaîtront plus clairement à la lecture de la description détaillée qui suit d'un mode de réalisation de l'invention donné à titre d'exemple nullement limitatif et illustré par les dessins annexés, dans lesquels :

- la figure 1 représente un schéma de principe montrant un volant équipé de deux électrodes pour détecter la présence d'un conducteur sur le volant, selon un premier exemple de réalisation particulier de l'invention ;
- la figure 2 représente un schéma électrique d'un dispositif capacitif de détection selon une première forme de réalisation de l'invention ;
- les figures 3, 4 et 5 représentent un plan complexe, en l'espèce un plan complexe d'admittance, comportant un repère orthonormé $(O, \vec{I}, \vec{J})$ sur lequel sont représentés des points de mesures brutes (non corrigées), des points de mesures corrigées et des points calculés ;

- la figure 6.1 représente un organigramme d'étapes d'une opération initiale de calibration ;
- la figure 6.2 représente un organigramme d'étapes d'une opération de mesure d'une valeur complexe recherchée, d'admittance ou d'impédance, entre une électrode de détection et la masse électrique du véhicule ;
- la figure 7 représente une séquence de mesures de la valeur complexe recherchée entrelacée avec des mesures de valeurs complexes (d'impédance ou d'admittance) de deux résistances de calibration représentées sur la figure 2.

L'invention est décrite ci-après, à titre d'exemple illustratif, dans le cadre d'un dispositif capacitif de détection monté sur le volant d'un véhicule, ledit dispositif étant destiné à détecter la présence d'un conducteur sur le volant.

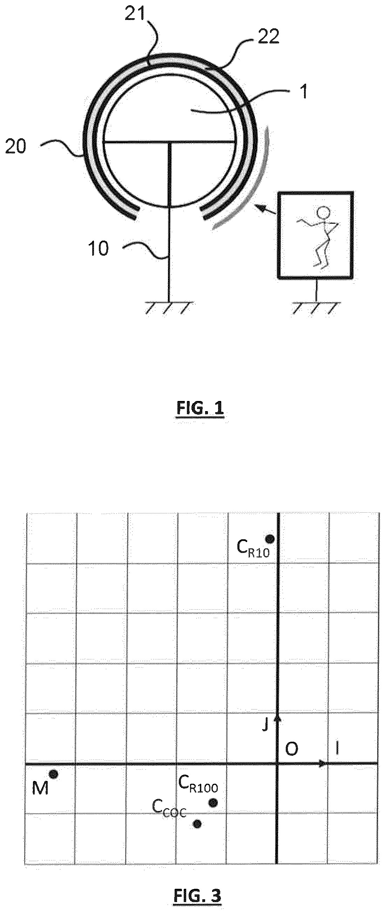

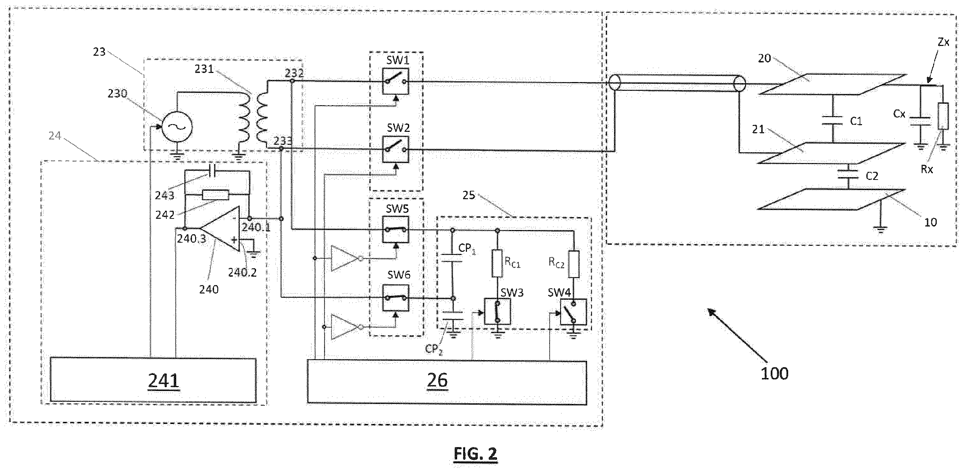

La figure 1 représente schématiquement un volant 1 muni d'une armature 10 qui peut être connectée à une masse électrique du véhicule. Selon l'invention, le volant est équipé d'une électrode externe 20, dite « électrode de détection », et d'une électrode interne 21, dite « électrode écran », séparées par une couche de matériau diélectrique 22. L'électrode 21 est présente entre l'électrode 20 et l'armature 10 du volant 1. L'électrode 21 est présente sur le volant et n'est pas en contact direct (c'est-à-dire en contact électrique direct) avec l'armature du volant. Lesdites électrodes 20, 21 et le matériau diélectrique forment un élément ou capteur capacitif ayant une capacité C1 de construction prédéterminée. La capacité C1 est généralement comprise entre 500 pF et 10 nF, de préférence entre 1 nF et 5 nF et encore plus préférentiellement entre 1 et 3 nF. Cette capacité est visible sur la figure 2.

Lorsque le conducteur du véhicule touche ou rapproche ses mains ou un doigt du volant, il vient ajouter une impédance complexe $Z_x$, correspondant à une capacité $C_x$ et une résistance $R_x$ disposées en parallèle, entre l'électrode de détection 20 et la masse électrique du véhicule. Cette impédance complexe $Z_x$ est fonction de la surface de l'organe approchée. Sa détermination permet par conséquent de détecter la présence ou l'absence d'un conducteur sur le volant (ou à proximité de celui-ci).

Sur la figure 2, on a représenté un dispositif capacitif de détection 100 selon un exemple particulier de réalisation de l'invention. Ce dispositif 100 comporte :

- l'électrode de détection 20 couplée à l'électrode écran 21,
- une source de tension 23 agencée pour délivrer une tension alternative,
- un dispositif 24 de mesure d'une valeur complexe d'impédance ou d'admittance,
- un dispositif de calibration 25 comportant au moins une résistance de calibration, ici deux résistances de calibration $R_{C1}$, $R_{C2}$ et
- un dispositif de commutation 26, SW1-SW6.

La figure 2 montre également différentes capacités présentes au niveau du volant 1 :

- la capacité de construction C1 présente entre les électrodes 20 et 21 ;
- une capacité de construction C2 présente entre l'électrode écran 21 et l'armature du volant 10, ici reliée à la masse électrique du véhicule ; et
- une capacité inconnue (à déterminer) $C_x$ présente ici entre l'électrode de détection 20 du volant 1 et la masse électrique du véhicule ; cette capacité $C_x$ inclut la capacité rapportée par les mains ou doigt(s) du conducteur sur le volant 1 et, le cas échéant, une ou plusieurs capacités de fuite liées à la construction du volant 1 ; elle représente la partie capacitive de l'impédance complexe $Z_x$ recherchée entre l'électrode de détection 20 et la masse électrique.

La source de tension 23 comporte un générateur de tension alternative 230 qui est ici connecté à un transformateur de tension 231. Le générateur de tension 230 est relié au primaire du transformateur 231. Le secondaire du transformateur 231 est relié à deux bornes de sortie 232 et 233 pour connecter la source de tension 23 à un composant externe à alimenter par un signal de tension alternative, via le transformateur 231. Le transformateur 231 crée une isolation galvanique entre le générateur de tension 230 et le composant externe connecté, et empêche la propagation d'effets de mode commun vers le composant connecté. En variante, le transformateur de tension pourrait être supprimé. Le dispositif de commutation comprend ici différents relais ou commutateurs électriques SW1 à SW6 et une unité de contrôle 26. D'une manière générale, sur commande de l'unité de contrôle 26, les relais sont agencés pour

- connecter la source de tension 23 à l'une au moins des électrodes 20 ou 21, en mode mesure,
- connecter la source de tension 23 à au moins une résistance de calibration $R_{C1}$, $R_{C2}$ et déconnecter la ou les électrodes 20 ou 21 de la source de tension 23, en mode calibration.

Dans l'exemple de réalisation décrit ici, les relais SW1 à SW6 ont plus précisément pour rôle de connecter la source de tension 23 de façon sélective

- soit à l'électrode de détection 20, en mode mesure ;

- soit au dispositif de calibration 25, en mode calibration.

L'ouverture et la fermeture des différents relais SW1 à SW6 sont commandées par l'unité de contrôle 26 pour obtenir les configurations électriques mentionnées ci-dessus en mode mesure et en mode calibration. Notamment, en mode calibration, la source de tension 23 doit être connectée au dispositif de calibration 25 et déconnectée de l'électrode (ici de l'électrode de détection 20) ; SW1 et SW2 sont ouverts et SW5 et SW6 sont fermés. Dans l'exemple particulier décrit ici, en mode mesure, la source de tension 23 est connectée à l'électrode (ici l'électrode de détection 20) et déconnectée du dispositif de calibration 25 ; SW1 et SW2 sont fermés et SW5 et SW6 sont ouverts. Toutefois, on pourrait envisager de maintenir la connexion entre la source de tension 23 et le dispositif de calibration 25 en mode mesure.

Par les termes « mode mesure », on entend désigner une configuration électrique du dispositif capacitif de détection 100 adaptée pour mesurer la valeur complexe d'impédance ou d'admittance entre l'une des électrodes, par exemple l'électrode de détection 20, et un point de référence de circuit électrique. Ce point de référence de circuit électrique est par exemple la masse électrique du véhicule. En variante, le point de référence pourrait être un autre point de circuit électrique, par exemple l'autre électrode du dispositif capacitif.

Par les termes « mode calibration », on entend désigner une configuration électrique du dispositif capacitif de détection 100 adaptée pour mesurer la valeur complexe d'impédance ou d'admittance d'une ou plusieurs résistances de calibration (ici $R_{C1}$ ou $R_{C2}$ ou éventuellement une résistance infinie équivalente à un circuit ouvert, comme expliqué plus loin).

L'unité de contrôle 26 est par exemple un microcontrôleur ou MCU (de l'anglais « *microcontroller unit* »).

Dans l'exemple particulier de réalisation décrit ici, l'électrode de détection 20 est connectée à la source de tension 23 par l'intermédiaire du relais SW1. Plus précisément, dans l'exemple de réalisation décrit ici en référence à la figure 2, l'électrode de détection 20 est connectée à la borne 232 du secondaire du transformateur 231 par l'intermédiaire du relais SW1. L'électrode écran 21 est reliée à la borne 233 du secondaire du transformateur 231 par l'intermédiaire du relais SW2. En mode mesure, les deux relais SW1 et SW2 sont fermés pour connecter les bornes de sortie 232 et 233 du transformateur 231 à l'électrode de détection 20 et à l'électrode écran 21 respectivement. En mode calibration, les deux relais SW1 et SW2 sont ouverts pour déconnecter la source de tension 23 des électrodes 20 et 21.

Le dispositif de mesure 24 est agencé pour mesurer une valeur complexe, correspondant à une impédance ou une admittance, entre une électrode et un point de référence de circuit électrique. Par exemple, le dispositif de mesure 24 est destiné à mesurer une impédance complexe recherchée « $Z_x$ » (ou une admittance $1/Z_x$) entre l'électrode de détection 20 et la masse électrique du véhicule. Cette impédance complexe $Z_x$ (ou admittance $1/Z_x$) est une impédance (ou admittance) inconnue, à déterminer de manière à détecter la présence ou l'absence des mains d'une personne sur le volant. Lorsqu'une personne, typiquement un conducteur, a les mains (ou une seule main ou bien un doigt ou plusieurs doigts sur le volant), l'impédance complexe $Z_x$ comporte une partie réelle de résistance $R_x$ et une partie imaginaire de capacitance $C_x$. En variante, l'admittance complexe $1/Z_x$ comporte une partie réelle de conductance et une partie imaginaire de susceptance.

Le dispositif de mesure 24 comporte ici un circuit de mesure de courant 240, 242-243 et un circuit de traitement 241.

Le circuit de mesure de courant 240, 242-243 comporte un amplificateur opérationnel 240 ayant une borne d'entrée négative (-) 240.1, une borne d'entrée positive (+) 240.2 et une borne de sortie 240.3. La borne d'entrée positive (+) 240.2 est connectée à une masse électrique ou à un signal de tension équivalent. La borne d'entrée négative 240.1 est destinée à recevoir un courant électrique à mesurer aux fins de mesurer une impédance ou admittance complexe. La borne de sortie 240.3 de l'amplificateur opérationnel 240 est reliée à la borne d'entrée négative 240.1 via une résistance 242 montée en parallèle avec un condensateur 243 de manière à ce que l'amplificateur opérationnel 240 forme un convertisseur courant-tension filtré par le condensateur 243.

La valeur de la résistance 242 est par exemple comprise entre 1 kΩ et 10 kΩ, préférentiellement entre 1 kΩ et 5 kΩ. La valeur de la résistance est avantageusement sélectionnée pour que les tests de compatibilité électromagnétique ne saturent pas l'étage d'entrée de l'amplificateur. La capacité du condensateur 243 est sélectionnée en fonction de la bande passante souhaitée par rapport à la fréquence du générateur de tension sinusoïdale. Par exemple, pour une fréquence 100kHz et une résistance 242 de 2kΩ, la capacité du condensateur 243 est de l'ordre de 220pF.

La tension entre les bornes d'entrée de l'amplificateur opérationnel 240 est nulle ou quasi-nulle et son impédance d'entrée est très élevée et considérée comme infinie. De ce fait, l'électrode écran 21 constitue une masse virtuelle, ce qui annule tout impact de la capacité C2 (entre l'électrode écran 21 et la masse électrique du véhicule) sur la mesure car la tension aux bornes de la capacité C2 est nulle.

L'amplificateur opérationnel 240 étant monté en convertisseur courant-tension, la tension présente à sa sortie est représentative du courant circulant à travers la borne d'entrée négative 240.1 de l'amplificateur opérationnel 240. En d'autres termes, la tension de sortie de l'amplificateur 240 est une réplique du courant circulant à travers la borne d'entrée négative 240.1.

Le circuit de traitement 241 est agencé pour déterminer une valeur complexe d'impédance ou d'admittance à partir de la tension de sortie de l'amplificateur opérationnel 240, représentative du courant circulant à travers la borne d'entrée 240.1. Il permet de déterminer l'impédance complexe $Z_x$ (ou l'admittance complexe $1/Z_x$), ici entre l'électrode de détection 20 et la masse électrique du véhicule, ainsi que l'impédance complexe (ou l'admittance complexe) d'une ou plusieurs résistances de calibration (ici $R_{C1}$, $R_{C2}$ ou éventuellement résistance infinie).

Une mesure de valeur complexe d'impédance ou d'admittance peut être représentée dans un plan complexe tel que celui représenté sur la figure 3. Cette figure 3 montre, à titre d'exemple illustratif, un plan complexe d'admittance comportant un repère orthonormé $(O, \vec{I}, \vec{J})$ dans lequel $\vec{I}$ et $\vec{J}$ représentent respectivement un vecteur définissant un axe des conductances $1/R$ et un vecteur définissant un axe des susceptances $C \times 2\pi \times f$ (où f représente la fréquence générée en Hertz par le DDS (de l'anglais « Direct Digital Synthesis », synthèse numérique directe) du circuit de traitement 241).

L'électronique de mesure (source de tension, transformateur, amplificateur AOP, etc.), les câbles de connexion et les conditions ambiantes, notamment la température, ont des effets perturbateurs sur la mesure d'impédance complexe. Afin de compenser ces effets perturbateurs ou les variations de performances liées aux variabilités des composants, le dispositif capacitif de détection 100 est agencé pour calibrer ou corriger une mesure de valeur complexe d'impédance ou d'admittance réalisée lors du fonctionnement du dispositif 100 en mode mesure à l'aide du dispositif de calibration 25.

Le dispositif de calibration 25 comprend ici deux résistances de calibration $R_{c1}$ et $R_{c2}$. Dans l'exemple de réalisation particulier décrit ici, les deux résistances de calibration $R_{c1}$ et $R_{c2}$ sont différentes l'une de l'autre.

Les résistances de calibration $R_{c1}$ et $R_{c2}$ sont avantageusement calibrées, c'est-à-dire dimensionnées, et ont des valeurs résistives déterminées pour garantir une calibration précise de la mesure dans des plages de valeurs pertinentes pour mesurer l'impédance ou l'admittance d'au moins une main ou d'au moins un doigt sur le volant. Par exemple, les résistances de calibrations $R_{c1}$ et $R_{c2}$ sont dimensionnées pour correspondre respectivement à deux mains posées sur le volant et à un doigt posé sur le volant. Autrement dit, la résistance de calibration $R_{c1}$ est dimensionnée pour être sensiblement égale (par approximation) à une impédance théorique ajoutée par deux mains posées sur le volant, constituée d'une capacité $C_{150}$ de l'ordre de 150 pF. Pour une fréquence arbitraire f de 100 kHz, cette impédance théorique $C_{150}$ $Z_{150pF}$ est déterminée par la relation suivante :

$$Z_{150pF} = \frac{1}{C_{150}.2\pi f} = 10{,}610 \ kohms$$

. Cette valeur théorique étant arrondie à 10 kilo-ohms, la résistance de calibration $R_{c1}$ choisie est ici une résistance de précision de 10K (10 kilo-ohms). De manière analogue, la résistance de calibration $R_{c2}$ est dimensionnée pour être sensiblement égale (par approximation) à l'impédance théorique ajoutée par un doigt posé sur le volant, constituée d'une capacité $C_{15}$ de l'ordre de 15 pF. Pour une fréquence arbitraire f de 100 kHz, cette impédance théorique $Z_{15pF}$ est déterminée par la relation suivante :

$$Z_{15pF} = \frac{1}{C_{15}.2\pi f} = 106{,}10 \ kohms$$

. Cette valeur théorique étant arrondie à 100K (100 kilo-ohms), la résistance de calibration $R_{c2}$ choisie est ici une résistance de précision de 100K (100 kilo-ohms). Les valeurs des résistances de calibration $R_{c1}$ et $R_{c2}$ peuvent toutefois être différentes de 10K et 100K, avantageusement comprises entre 1K et 200K, préférentiellement entre 5K et 150K. La valeur de la résistance $R_{c1}$ est par exemple comprise entre 1 k$\Omega$ et 20 k$\Omega$, préférentiellement entre 5 k$\Omega$ et 15 k$\Omega$. La valeur de la résistance $R_{c2}$ est par exemple comprise entre 10 k$\Omega$ et 200 k$\Omega$, préférentiellement entre 50 k$\Omega$ et 150 k$\Omega$. Les résistances de calibration permettent de créer des référentiels pour la mesure d'une valeur complexe d'impédance ou d'admittance. De préférence, des résistances de précision sont utilisées afin d'obtenir une très faible dérive en température (25 ppm), une très bonne tolérance (0,1%), une meilleure tenue aux contraintes mécaniques, une meilleure résistance à la moisissure et en fabrication durant un passage au four. Par exemple, les résistances de calibration sont obtenues par la technologie « thin film ».

Les deux résistances de calibration $R_{c1}$ et $R_{c2}$ sont ici montées en parallèle. Chacune des résistances $R_{c1}$ ($R_{c2}$) est connectée comme suit, au niveau de ses deux bornes respectivement :

- à la source de tension 23, via un commutateur ou relais électrique SW5 commun; plus précisément dans l'exemple de réalisation décrit ici en référence à la figure 2, les deux résistances $R_{c1}$ ($R_{c2}$) sont connectées à la borne de sortie 232 du transformateur 231 via le relais SW5 ;

- à la masse électrique du véhicule, par l'intermédiaire d'un commutateur ou relais électrique SW3 (SW4) individuel.

Dans la forme particulière de réalisation décrite ici, le dispositif de calibration 25 comprend également une paire de deux capacités (ou condensateurs) de protection $C_{P1}$ et $C_{P2}$ montées en série. La paire de capacités $C_{P1}$ et $C_{P2}$ en série est montée en parallèle des résistances de calibration $R_{c1}$ et $R_{c2}$ et connectée à la masse électrique du véhicule. Ces capacités $C_{P1}$ et $C_{P2}$ permettent de protéger les résistances de calibration, qui sont ici des résistances de précision plus sensibles que les résistances classiques, des effets d'ESD (de l'anglais « *electrostatic discharge* ») de décharge électrostatique. Elles sont dimensionnées dans des gammes de capacités proches des capacités de construction C1 et C2. Plus précisément, la capacité $C_{P1}$ est de l'ordre de la capacité C1 et la capacité $C_{P2}$ est de l'ordre de la capacité C2. Plus précisément, $C_{P1}$ est comprise entre 500 pF et 500 nF, préférentiellement égale à 10 nF, et $C_{P2}$ est comprise entre 100 pF et 10 nF, préférentiellement égale à 1 nF. Grâce à cela, le courant mesuré lors d'une calibration est similaire à un courant mesuré lors d'une mesure de l'impédance $Z_x$ (ou admittance $1/Z_x$) recherchée. Il en résulte une précision de mesure optimale.

La résistance de calibration $R_{c1}$ couplée en série avec le relais SW3, la résistance de calibration $R_{c2}$ couplée en série avec le relais SW4 et la paire de capacités $C_{P1}$ et $C_{P2}$ en série forment trois branches de circuit connectées en parallèle entre la source de tension alternative 23 (en l'espèce la borne de sortie 232 du transformateur 231), via le relais SW5, et la masse électrique du véhicule.

Le dispositif de commutation comprend également un relais SW6 interposé entre la borne de sortie 233 du transformateur 231 et un point de raccordement entre les deux capacités de protection $C_{P1}$ et $C_{P2}$. Ainsi, en mode calibration, la capacité de protection $C_{P1}$ est reliée à ses bornes aux deux bornes de sortie 232 et 233 du secondaire du transformateur 231, via le premier relais SW5 et le deuxième relais SW6 respectivement. La capacité de protection $C_{P2}$ est reliée à ses deux bornes à la borne de sortie 233 du secondaire du transformateur 231 et à la masse électrique, respectivement, par l'intermédiaire du relais SW6.

Le circuit de mesure 24 est destiné à recevoir un signal de courant représentatif d'une impédance ou d'une admittance à mesurer ici à travers la borne d'entrée négative 240.1 de l'amplificateur 240. A cet effet, le dispositif de commutation est agencé pour connecter la borne d'entrée 240.1 soit à la branche reliant la borne de sortie 233 de la source de courant 23, en mode mesure, soit au point de raccordement entre les deux capacités de protection $C_{P1}$ et $C_{P2}$ via le relais SW6. Dans l'exemple de réalisation de la figure 2, la borne d'entrée négative 240.1 de l'amplificateur 240 est connectée à une branche de connexion reliant le relais SW6 et la borne de sortie 233 du transformateur 231. Dans le cas où les capacité $C_{P1}$ et $C_{P2}$ ne seraient pas utilisées, la borne d'entrée négative 240.1 de l'amplificateur 240 serait connectée uniquement à la borne de sortie 233 du transformateur 231, les résistance $R_{C1}$ et $R_{C2}$ seraient quant à elles directement connectées à la borne de sortie 232 du transformateur 231.

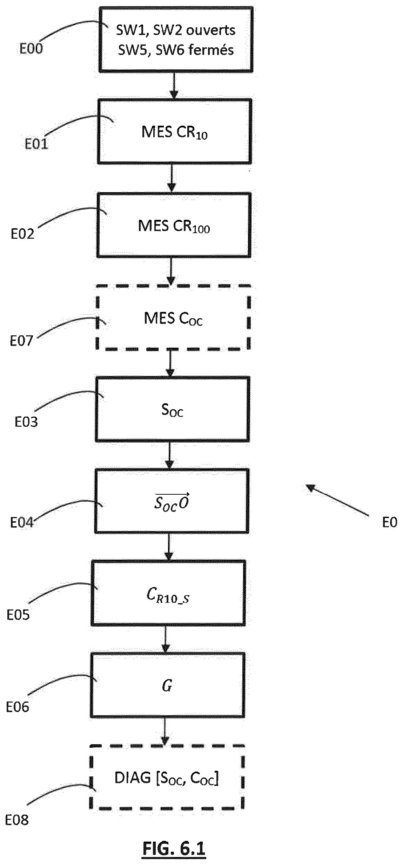

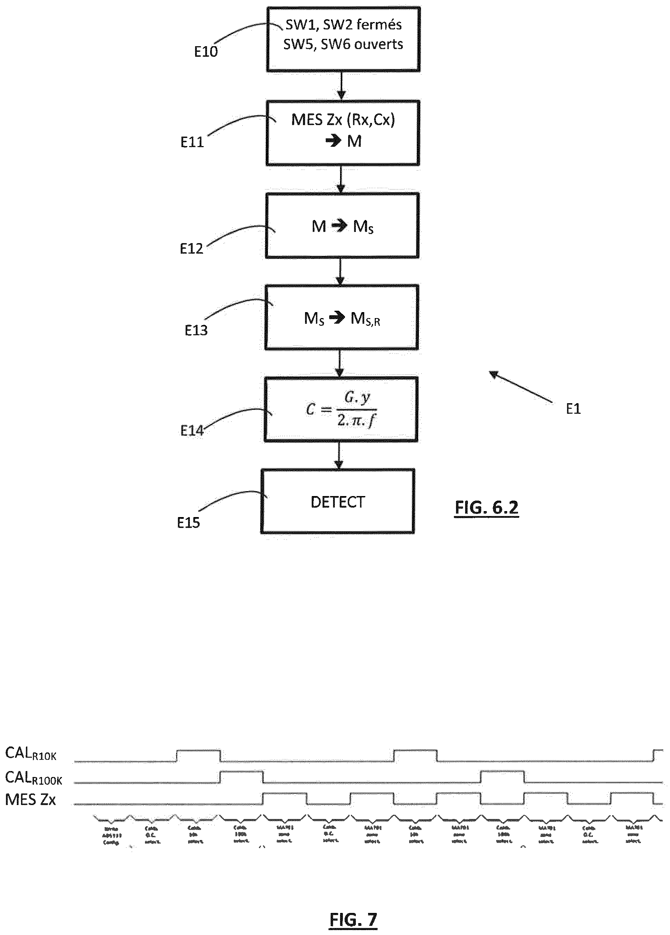

On va maintenant décrire un procédé de détection d'une personne sur le volant 1 du véhicule, correspondant au fonctionnement du dispositif capacitif de calibration 100, en référence aux graphes des figures 3 à 5 et aux organigrammes des figures 6.1 et 6.2.

La présente invention permet de calibrer (ou corriger) des mesures de valeurs complexes d'impédance ou d'admittance ici entre l'électrode de détection 20 et la masse électrique à partir des résistances de calibration $R_{c1}$ et $R_{c2}$ ayant des valeurs résistives connues et ici différentes l'une de l'autre. Les résistances de calibration $R_{c1}$ et $R_{c2}$ sont utilisées pour réaliser des mesures de calibration. Ces mesures de calibration peuvent être réitérées pour mettre à jour la calibration de façon continue, comme cela sera décrit par la suite.

Le procédé de détection comprend une opération initiale de calibration E0, représentée sur la figure 6.1. Cette opération initiale de calibration E0 vise à mesurer la valeur complexe d'impédance ou d'admittance de chacune des résistances de calibration et également ici une valeur complexe d'impédance ou d'admittance en circuit ouvert équivalente à l'impédance ou admittance complexe d'une résistance infinie. Elle comprend une première étape E00 de configuration en mode calibration du dispositif capacitif de détection 100. Lors de cette étape E00, sur commande de l'unité de contrôle 26, les relais SW1 et SW2 sont ouverts et les relais SW5 et SW6 sont fermés. La source de tension 23 est alors déconnectée des électrodes 20, 21 et connectée au dispositif de calibration 25 de manière à alimenter en tension alternative l'étage de calibration ici à travers la paire de capacités de protection $C_{P1}$ et $C_{P2}$.

Dans cette configuration du dispositif de détection 100 (SW1-SW2 ouverts et SW5-SW6 fermés), les résistances de calibration $R_{c1}$ et $R_{c2}$ sont connectées l'une après l'autre (séparément) à la source de tension 23 pour réaliser deux mesures de valeur complexe d'impédance ou d'admittance respectivement de la résistance $R_{c1}$ et de la résistance $R_{c2}$. Ainsi, le dispositif de commutation est agencé pour connecter la source de tension 23 de façon sélective à une première des deux résistances $R_{c1}$, puis à une deuxième des deux résistances $R_{c2}$. Plus précisément, le relais SW3 étant fermé et le relais SW4 ouvert, le dispositif de mesure 24 mesure l'impédance ou l'admittance complexe de la résistance de calibration $R_{c1}$, lors d'une première étape de mesure de calibration E01. Puis, le relais SW3 étant ouvert et le relais SW4 fermé, le dispositif de mesure 24 mesure l'impédance ou l'admittance complexe de la résistance de calibration $R_{c2}$, lors d'une deuxième sous-étape de mesure de calibration E02. Sur les figures 3 à 5, les points $C_{R10}$ et $C_{R100}$ représentent, dans le plan complexe ici d'admittance $(O, \vec{I}, \vec{J})$, respectivement la mesure brute de valeur complexe d'admittance de la résistance de calibration $R_{C1}$ et la mesure brute de valeur complexe d'admittance de la résistance de calibration $R_{C2}$. Par « mesure brute », on entend désigner une mesure non corrigée. Les étapes E01 et E02 peuvent être exécutées dans un ordre quelconque.

Lors d'une étape suivante E03, le circuit de traitement 241 calcule ici l'admittance en circuit ouvert théorique Soc du dispositif de détection 100, autrement dit l'admittance d'une résistance de calibration infinie (remplaçant par exemple $R_{c1}$ ou $R_{c2}$), à partir des mesures de valeur complexe (d'impédance ou d'admittance) des deux résistances de calibration $R_{c1}$ et $R_{c2}$, selon la relation suivante :

$$\overrightarrow{S_{OC}C_{R10}} = 10 \times \overrightarrow{S_{OC}C_{R100}} \tag{1}$$

La relation (1) traduit les propriétés mathématiques suivantes :

- les admittances Soc, $C_{R10}$ et $C_{R100}$ sont alignées dans le plan complexe d'admittance car toutes les trois représentent uniquement des résistances et

- il existe un facteur 10 de proportionnalité entre l'amplitude de l'admittance $C_{R10}$ par rapport à l'admittance en circuit ouvert Soc et l'amplitude de l'admittance $C_{R100}$ par rapport à l'admittance en circuit ouvert Soc.

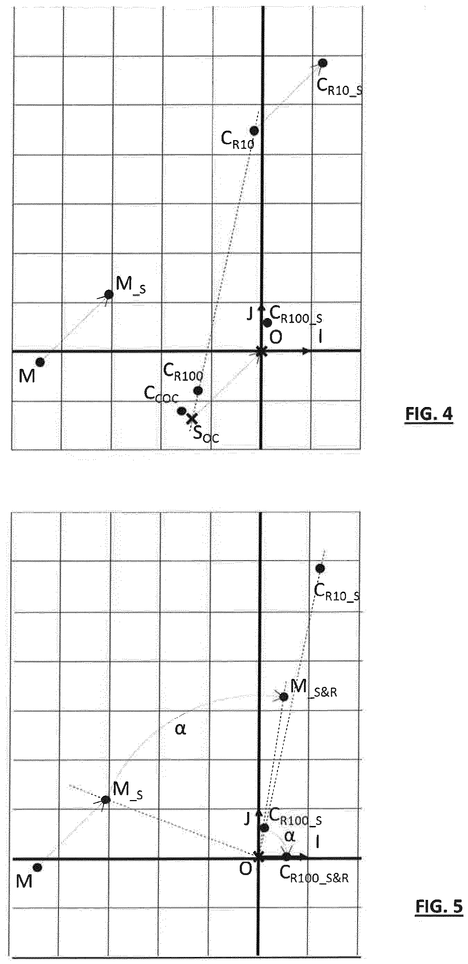

Dans le plan complexe $(O,\vec{I},\vec{J})$, par définition, on considère que le point Soc d'impédance infinie théorique est l'origine O du repère orthonormé. Or, comme le montrent les figures 3 et 4, en raison des effets perturbateurs précédemment évoqués, le point Soc et l'origine O du repère $(O,\vec{I},\vec{J})$ ne coïncident pas dans le plan complexe $(O,\vec{I},\vec{J})$. La translation qui envoie le point Soc sur l'origine O du plan complexe $(O,\vec{I},\vec{J})$ définit un vecteur $\overrightarrow{S_{OC}O}$. La translation caractérisée par le vecteur $\overrightarrow{S_{OC}O}$ définit une première correction par décalage ou « correction d'offset » à appliquer à une mesure faite par le dispositif de mesure 24. Lors d'une étape E04, le circuit de traitement 241 calcule le vecteur d'offset ou de translation ou de décalage $\overrightarrow{S_{OC}O}$.

Lors d'une étape E05, le circuit de traitement 241 applique sur l'une au moins des deux mesures de valeur complexe d'impédance ou d'admittance des résistances $R_{c1}$ et $R_{c2}$ une première correction correspondant à une translation selon le vecteur $\overrightarrow{S_{OC}O}$ du point $C_{R10}$ (ou $C_{R100}$) dans le plan complexe $(O,\vec{I},\vec{J})$. Le point image du point $C_{R10}$ (ou $C_{R100}$) par la translation selon le vecteur $\overrightarrow{S_{OC}O}$ est un point décalé $C_{R10\_S}$ (ou $C_{R100\_S}$) dans le plan complexe $(O,\vec{I},\vec{J})$, comme représenté sur la figure 4. Il satisfait la relation suivante :

$$\overrightarrow{C_{R10}C_{R10\_S}} = \overrightarrow{S_{OC}O} \tag{2}$$

Lors d'une étape E06, le circuit de traitement 241 calcule un gain G de conversion en unités de mesure standard, respectivement siemens et farad pour les conductances et les capacités. Le gain de conversion G est calculé selon la relation suivante :

$$G = \frac{|R_{100\Omega}-R_{10\Omega}|}{R_{100\Omega}\times R_{10\Omega}\times\|\overrightarrow{C_{R10}C_{R100}}\|} = \frac{9.10^4}{10^9\times\|\overrightarrow{C_{R10}C_{R100}}\|} = \frac{9}{10^5\times\|\overrightarrow{C_{R10}C_{R100}}\|} \tag{3}$$

La relation (3) ci-dessus découle du fait que la différence entre les admittances respectives des deux résistances de calibration $R_{c1}$ et $R_{c2}$ est égale au gain G multiplié par la norme du vecteur reliant le point de mesure $C_{R10}$ et le point

$$\frac{1}{R_{10\Omega}} - \frac{1}{R_{100\Omega}} = G \times \|\overrightarrow{C_{R10}C_{R100}}\|$$

de mesure $C_{R100}$. Autrement dit :

Le procédé peut également comprendre une mesure d'une valeur complexe d'impédance ou d'admittance en circuit ouvert, réalisée par exemple pendant l'opération initiale de calibration E0 après les mesures E01 et E02 d'impédance des résistances de calibration $R_{c1}$ et $R_{c2}$, lors d'une étape E07 (représentée en pointillés sur la figure 6.1). Cette valeur complexe d'impédance ou d'admittance en circuit ouvert correspond à l'impédance ou admittance d'une résistance de calibration mais de valeur résistive infinie. Notons Coc, le point du plan d'admittance $(O,\vec{I},\vec{J})$ correspondant à la mesure de valeur complexe d'admittance en circuit ouvert, comme représentés sur les figures 3 et 4. Lors d'un bon fonctionnement du dispositif de détection 100, le point Coc est proche du point théorique calculé Soc, comme le montre la figure 4. Un décalage trop important entre Coc et Soc indique un dysfonctionnement du dispositif capacitif de détection 100. Le procédé comprend de façon optionnelle une étape E08 de diagnostic de bon fonctionnement, lors de laquelle le circuit de traitement 241 vérifie si le point mesuré Coc est proche du point théorique calculé Soc afin de détecter le bon ou mauvais fonctionnement du dispositif de détection 100. Par exemple, la distance entre les deux points Coc et Soc est calculée et comparée à une valeur limite (ou seuil). Si la distance calculée est inférieure à la valeur limite, on considère que le fonctionnement est bon. Si la distance calculée est supérieure à la valeur limite, on détecte un dysfonctionnement du dispositif de détection 100. Dans ce cas, un signal d'alerte peut être transmis au conducteur du véhicule.

L'opération de calibration E0 est suivie d'une opération E1 de mesure de l'impédance entre l'électrode de détection 20 et la masse électrique du véhicule. Lorsqu'un conducteur a les mains sur le volant (ou bien seulement un doigt ou quelques doigts), il ajoute une impédance $Z_x$ comportant une résistance $R_x$ et une capacité $C_x$ en parallèle entre l'électrode de détection 20 et la masse électrique, comme représenté sur la figure 2.

L'opération de mesure E1 comprend une première étape E10 de configuration en mode mesure du dispositif capacitif de détection 100. Lors de cette étape E10, sur commande de l'unité de contrôle 26, les relais SW1 et SW2 sont fermés et les relais SW5 et SW6 sont ici ouverts. La source de tension 23 est alors connectée aux électrodes 20, 21, l'électrode de détection 20 étant connectée à la borne de sortie 232 de la source de tension, et déconnectée du dispositif de calibration 25 de manière à alimenter en tension alternative l'électrode de détection 20. Dans cette configuration, les relais SW3 et SW4 sont avantageusement ouverts.

Lors d'une étape de mesure E11, l'électrode de détection 20 reçoit un signal de tension alternative et le circuit de mesure 24 mesure le courant électrique circulant à travers l'électrode écran 21 à l'aide de l'amplificateur 240. Le circuit de traitement 241 détermine une valeur complexe d'impédance $Z_x$ ou d'admittance $1/Z_x$ entre l'électrode de détection 20 et la masse. Sur la figure 4, on a représenté ici dans le plan complexe d'admittance $(O,\vec{I},\vec{J})$ un point de mesure M représentant la mesure de valeur complexe d'impédance $Z_x$ ou d'admittance $1/Z_x$ ainsi que le point de mesure décalé $C_{R10\_S}$ de la résistance $R_{C1}$, l'origine O et le point calculé Soc correspondant à une impédance infinie (ou en circuit ouvert).

Lors d'une étape suivante E12, le circuit de traitement 241 applique à la mesure de la valeur complexe d'impédance $Z_x$ ou d'admittance $1/Z_x$ une première correction correspondant à une translation par le vecteur $\overrightarrow{S_{OC}O}$ du point de mesure M dans le plan d'admittance $(O,\vec{I},\vec{J})$. Le point image du point de mesure M par cette translation est un point décalé Ms dans le plan d'admittance $(O,\vec{I},\vec{J})$. Il satisfait la relation suivante :

$$\overrightarrow{MM_S} = \overrightarrow{S_{OC}O} \tag{4}$$

Les résistances de calibration $R_{C1}$, $R_{C2}$ sont des résistances pures ayant des valeurs (résistives) réelles. Par convention, les résistances sont représentées dans le plan complexe ici d'admittance $(O,\vec{I},\vec{J})$ par des points sur l'axe des conductances $(O,\vec{I})$. Sur la base de cette propriété géométrique et d'au moins deux des points représentant les valeurs complexes mesurées des résistances $R_{C1}$, $R_{C2}$ et la valeur complexe d'impédance ou d'admittance en circuit ouvert calculée (à savoir $C_{R10}$, $C_{R100}$ et $S_{OC}$ avant correction par décalage ou bien $C_{R10\_S}$, $C_{R100\_S}$ et O après décalage par $\overrightarrow{S_{OC}O}$), le circuit de traitement 241 applique au point de mesure décalé Ms une correction de phase dans le plan complexe lors d'une étape E13, par exemple par les relations suivantes :

$$\left\| \overrightarrow{OM_S} \right\| = \left\| \overrightarrow{OM_{S\&R}} \right\| \tag{5}$$

$$\alpha = \left( \overrightarrow{OM_S}, \overrightarrow{OM_{S\&R}} \right) = \left( \overrightarrow{OC_{R10,S}}, \overrightarrow{OI} \right) \tag{6}$$

où $M_{S\&R}$ représente le point de mesure M après correction d'offset et correction de phase.

D'une manière plus générale, ladite correction de phase correspond à une rotation selon un angle $\alpha$ entre :

- une droite définie par deux points du plan complexe (d'admittance $(O,\vec{I},\vec{J})$ ou d'impédance), obtenus à partir de mesures de valeurs complexes d'impédance ou d'admittance des résistances (résistance(s) de calibration et/ou résistance infinie) et
- l'axe $(O,\vec{I})$ des conductances ou des résistances du plan complexe.

Les deux points du plan complexe $(O,\vec{I},\vec{J})$ obtenus à partir de mesures de valeurs complexes d'impédance ou d'admittance de résistances peuvent être sélectionnés parmi l'un des deux groupes de points suivants :

- $C_{R10}$, $C_{R100}$ et Soc,

- $C_{R10\_S}$, $C_{R100\_S}$ et O,
- ou par exemple $C_{R10}$ et Coc

Le circuit de traitement détermine ainsi une valeur complexe (d'impédance ou d'admittance) corrigée par offset (ou décalage) et correction de phase, représentée ici par le point $M_{\_S\&R}$ dans le plan d'admittance $(O, \vec{I}, \vec{J})$. Notons x et y les coordonnées d'abscisse et d'ordonnée du point $M_{\_S\&R}$ dans le plan d'admittance $(O, \vec{I}, \vec{J})$.

La correction par décalage ou offset et la correction de phase peuvent être exécutées dans un ordre quelconque pour autant que les calculs sont adaptés.

Lors d'une étape de mise à l'échelle E14, l'unité de traitement 241 met à l'échelle les coordonnées x et y du point $M_{\_S\&R}$ pour les convertir en unités de mesure ohms et farads respectivement, selon les relations suivantes :

$$C = \frac{G.y}{2.\pi.f} \qquad (7)$$

$$R = \frac{1}{G.x} \qquad (8)$$

où f représente la fréquence en Hertz générée par le DDS (de l'anglais « Direct Digital Synthesis ») du circuit intégré de traitement 241.

Lors d'une étape finale E15, l'unité de traitement 241 détecte la présence ou l'absence d'un conducteur sur le volant 1 par exploitation de la valeur de capacité C obtenue lors de l'étape E14. Cette valeur de capacité C permet d'évaluer la surface de main ou doigt en contact avec le volant 1. Une capacité de l'ordre de 15 pF correspond à un doigt posé sur le volant 1. Une capacité de l'ordre de 150 pF correspond à deux mains posées sur le volant 1.

Dans un mode de réalisation particulier de l'invention, suite à l'exécution de l'opération initiale de calibration E0 suivie de l'opération E1 de mesure d'impédance $Z_x$, l'étape E11 de mesure d'une valeur complexe d'impédance $Z_x$ (ou d'admittance $1/Z_x$) est répétée de façon cyclique et, entre deux mesures de $Z_x$ (ou $1/Z_x$) des étapes de calibration (E01, E02 et/ou E07) sont répétées. Les mesures de l'impédance (ou admittance) recherchée $Z_x$ (ou $1/Z_x$) sont ainsi entrelacées avec des mesures de calibration pour garantir une calibration continue pendant toute la durée de la détection du volant 1. Par exemple, une moitié des mesures est dédiée à des mesures d'impédance $Z_x$ (ou d'admittance $1/Z_x$) en mode mesure et l'autre moitié des mesures est dédiée à des mesures de calibration (E01, E02 ou E07) en mode calibration pour mettre à jour les données de calibration au fur et à mesure et de façon continue dans le temps. Sur la figure 7, on a représenté un exemple de mise en oeuvre de cet entrelacement des mesures de $Z_x$ et des mesures de calibration. Les première et deuxième lignes représentent les mesures d'impédances des résistances $R_{C1}$ et $R_{C2}$ respectivement. La dernière ligne représente les mesures d'impédance $Z_x$ (ou d'admittance $1/Z_x$). Initialement, comme décrit en référence à la figure 6.1, le dispositif de détection 100 réalise une mesure de valeur complexe (d'impédance ou d'admittance) de la résistance $R_{C1}$ puis une mesure de valeur complexe (d'impédance ou d'admittance) de la résistance $R_{C2}$ afin d'obtenir un premier jeu de données de calibration. Ensuite une première mesure d'impédance $Z_x$ (ou d'admittance $1/Z_x$) est réalisée. Après cette phase initiale, les mesures d'impédance $Z_x$ (ou d'admittance $1/Z_x$) sont répétées de façon régulière, selon une fréquence de mesure déterminée, et, entre deux mesures, une seule mesure de calibration est ici réalisée, à savoir soit une mesure d'impédance de la résistance de calibration $R_{C1}$, soit une mesure d'impédance de la résistance de calibration $R_{C2}$. On pourrait également alterner ces mesures de calibration avec une mesure en circuit ouvert (équivalent à une résistance de calibration de valeur infinie). Ainsi, les différentes mesures de calibration sont exécutées l'une après l'autre de façon cyclique entre deux mesures d'impédance $Z_x$ (ou d'admittance $1/Z_x$) successives. Les mesures de calibration sont ainsi entrelacées entre les mesures d'impédance $Z_x$ (ou d'admittance $1/Z_x$).

L'entrelacement des mesures d'impédance $Z_x$ (ou d'admittance $1/Z_x$) et des mesures de calibration est avantageusement exécuté pendant toute la durée de fonctionnement le dispositif de détection 100, typiquement à partir du moment où le véhicule est mis en marche et jusqu'à l'arrêt de son moteur.

Dans la description qui précède, le dispositif capacitif de détection 100 comporte deux résistances de calibration ayant des valeurs résistives différentes l'une de l'autre.

Dans une variante de réalisation selon la revendication 3, le dispositif capacitif de détection comporte deux résistances de calibration semblables (c'est-à-dire ayant une même valeur résistive). Dans ce cas, des moyens de commutation sont prévus pour réaliser deux dipôles résistifs différents à partir des deux résistances de calibration, ce qui permet de réaliser deux mesures de valeur complexe d'impédance (ou d'admittance) et d'obtenir deux points distincts de mesure de calibration dans le plan complexe. Un premier dipôle résistif comprend l'une seule des deux résistances de calibration et le second dipôle résistif comprend les deux résistances de calibration montées en série.

Dans une variante de réalisation ne faisant pas partie de l'invention revendiquée, le dispositif capacitif de détection comprend une seule résistance de calibration permettant de réaliser une première étape de mesure d'impédance d'une résistance de calibration, par exemple comprise entre 10 kΩ et 100 KΩ. Dans ce cas, la deuxième étape de mesure d'impédance d'une autre résistance de calibration est supprimée et le procédé comprend une étape de mesure de la résistance en circuit ouvert, identique à l'étape E07, afin d'obtenir une mesure de l'impédance en circuit ouvert. Dans ce cas, les deux corrections (correction d'offset et correction de phase) à appliquer sont déterminées à partir du point représentant la mesure d'impédance d'une résistance de calibration dans le plan d'admittance, par exemple $C_{R100}$ (si la résistance de calibration est de 100 kΩ) et du point Coc représentant la mesure d'impédance en circuit ouvert. L'impédance (ou admittance) en circuit ouvert est dans ce cas mesurée mais n'est pas calculée de façon théorique (le point Soc n'est pas calculé).

Dans la description qui précède, l'électrode de détection est connectée à la source de tension 23, en l'espèce à sa borne de sortie 232, via le relais SW1. Dans une variante de réalisation du montage électrique, c'est l'électrode écran qui est connectée à la source de tension 23, en l'espèce à sa borne de sortie 232, via le relais SW1.

Dans la description qui précède, le dispositif de mesure 24 mesure une valeur complexe d'impédance ou d'admittance aux fins de détecter la présence ou l'absence d'une personne sur le volant entre une électrode (par exemple l'électrode de détection 20) et une masse électrique de véhicule. En variante, le dispositif de mesure d'impédance 24 mesure une valeur complexe d'impédance ou d'admittance aux fins de détecter la présence ou l'absence d'une personne sur le volant entre l'une des deux électrodes 20, 21 et un point de référence de circuit électrique. Le point de circuit électrique constituant un point de référence pour la mesure d'impédance ou d'admittance complexe recherchée peut être l'autre électrode.

La présente invention a été décrite dans le cadre de la détection de présence d'un conducteur sur le volant du véhicule. Dans un autre exemple de réalisation de l'invention, le dispositif capacitif de détection est destiné à détecter la présence ou l'absence d'une personne sur un siège du véhicule. De manière plus générale, l'invention s'applique à la détection de présence d'une personne à proximité ou sur (c'est-à-dire au contact de) un composant d'un véhicule automobile.

**Revendications**

**1.** Dispositif de détection capacitif (100) pour détecter la présence d'une personne à proximité ou au contact avec un composant (1) d'un véhicule automobile, équipé d'un capteur capacitif, ledit dispositif comprenant

- au moins une électrode (20) du capteur capacitif,
- une source de tension (23) agencée pour délivrer une tension alternative,
- un dispositif (24) de mesure d'une valeur complexe, correspondant à une impédance ou une admittance, entre ladite électrode (20) et un point de référence de circuit électrique;
- une première résistance de calibration ($R_{c1}$, $R_{c2}$) pour calibrer la mesure d'une valeur complexe d'impédance ou d'admittance ;

dans lequel :

ledit dispositif de détection capacitif comprend un dispositif de commutation (26, SW1-SW6) agencé pour

- connecter la source de tension (23) à ladite électrode (20), en mode mesure,
- connecter la source de tension (23) à la première résistance de calibration ($R_{c1}$, $R_{c2}$) et déconnecter ladite électrode (20) de la source de tension, en mode calibration,

et le dispositif (24) de mesure est agencé pour

- mesurer une première valeur complexe de la première résistance de calibration ($R_{C1}$, $R_{C2}$), lors d'un fonctionnement en mode calibration ;
- mesurer une deuxième valeur complexe entre ladite électrode (20) et ledit point de référence de circuit électrique, lors d'un fonctionnement en mode mesure, et
- corriger la deuxième valeur complexe mesurée en fonction de la première valeur complexe mesurée,

**caractérisé en ce que** le dispositif de détection capacitif (100) comprend en outre une deuxième résistance de calibration ($R_{C1}$, $R_{C2}$), **en ce que** la première et la deuxième résistance de calibration ($R_{C1}$, $R_{C2}$) ont des valeurs résistives différentes,

et **en ce que** les deux résistances de calibration ($R_{C1}$, $R_{C2}$) sont montées en parallèle et le dispositif de commutation (26, SW1-SW6) est agencé pour connecter la source de tension (23) de façon sélective à l'une ou l'autre des deux résistances ($R_{C1}$, $R_{C2}$).

2. Dispositif selon la revendication précédente, **caractérisé en ce que** la première résistance de calibration ($R_{C1}$) a une valeur résistive comprise entre 1 kΩ et 20 kΩ et la deuxième résistance de calibration ($R_{C2}$) a une valeur résistive comprise 10 kΩ et 200 KΩ.

3. Dispositif de détection capacitif (100) pour détecter la présence d'une personne à proximité ou au contact avec un composant (1) d'un véhicule automobile, équipé d'un capteur capacitif, ledit dispositif comprenant

   - au moins une électrode (20) du capteur capacitif,
   - une source de tension (23) agencée pour délivrer une tension alternative,
   - un dispositif (24) de mesure d'une valeur complexe, correspondant à une impédance ou une admittance, entre ladite électrode (20) et un point de référence de circuit électrique ;
   - une première résistance de calibration ($R_{c1}$, $R_{c2}$) pour calibrer la mesure d'une valeur complexe d'impédance ou d'admittance ;

   dans lequel :

   ledit dispositif de détection capacitif comprend un dispositif de commutation (26, SW1-SW6) agencé pour

   - connecter la source de tension (23) à ladite électrode (20), en mode mesure,
   - connecter la source de tension (23) à la première résistance de calibration ($R_{c1}$, $R_{c2}$) et déconnecter ladite électrode (20) de la source de tension, en mode calibration,

   et le dispositif (24) de mesure est agencé pour

   - mesurer une première valeur complexe de la première résistance de calibration ($R_{C1}$, $R_{C2}$), lors d'un fonctionnement en mode calibration ;
   - mesurer une deuxième valeur complexe entre ladite électrode (20) et ledit point de référence de circuit électrique, lors d'un fonctionnement en mode mesure, et
   - corriger la deuxième valeur complexe mesurée en fonction de la première valeur complexe mesurée,

   **caractérisé en ce que** le dispositif de détection capacitif (100) comprend en outre une deuxième résistance de calibration ($R_{C1}$, $R_{C2}$), ence que la première et la deuxième résistance de calibration ($R_{C1}$, $R_{C2}$) ont des valeurs identiques,
   et **en ce que** les deux résistances de calibration ($R_{C1}$, $R_{C2}$) sont montées en série et le dispositif de commutation (26, SW1-SW6) est agencé pour connecter la source de tension (23) de façon sélective à un premier dipôle résistif comprenant l'une seule des deux résistances de calibration ($R_{C1}$, $R_{C2}$) ou à un second dipôle résistif comprenant les deux résistances de calibration ($R_{C1}$, $R_{C2}$).

4. Dispositif selon l'une des revendications précédentes, **caractérisé en ce que** le dispositif de mesure (24) est agencé pour appliquer une correction d'offset à la deuxième valeur complexe mesurée, ladite correction d'offset correspondant à une translation selon un vecteur $\overrightarrow{S_{OC}O}$ dans un plan complexe, le point Soc étant un point du plan complexe correspondant à une valeur complexe d'admittance ou d'impédance en circuit ouvert du dispositif capacitif de détection et le point O correspondant à l'origine du plan complexe.

5. Dispositif selon la revendication précédente, **caractérisé en ce que** le dispositif de mesure (24) est agencé pour calculer ladite valeur complexe d'impédance ou d'admittance en circuit ouvert (SOC) du dispositif capacitif de détection à partir de deux valeurs complexes mesurées à l'aide des deux résistances de calibration ($R_{C1}$, $R_{C2}$).

6. Dispositif selon la revendication précédente, **caractérisé en ce que** le dispositif de mesure (24) est agencé pour mesurer une valeur complexe d'impédance ou d'admittance en circuit ouvert (COC) et comparer ladite valeur complexe d'impédance ou d'admittance en circuit ouvert mesurée et ladite valeur complexe d'impédance ou d'admittance en circuit ouvert calculée (SOC) afin de vérifier le bon fonctionnement du dispositif capacitif de détection.

**7.** Dispositif selon l'une des revendications 1 à 6, **caractérisé en ce que** le dispositif de mesure (24) est agencé pour appliquer une correction de phase (E13) à la deuxième valeur complexe mesurée, ladite correction de phase correspondant à une rotation selon un angle $\alpha$ entre une droite définie par deux points dans un plan complexe, obtenus à partir de deux valeurs complexes d'impédance ou d'admittance mesurées à l'aide des deux résistances de calibration, et un axe des résistances ou des conductances du plan complexe.

**8.** Dispositif selon l'une des revendications précédentes, **caractérisé en ce qu'**il comprend une paire de capacités de protection en série (CP1, CP2), ladite paire de capacités étant montée en parallèle des résistances de calibration ($R_{C1}$, $R_{C2}$) et **en ce que** l'une des deux capacités est reliée à ses bornes à la source de tension (23) par l'intermédiaire de deux commutateurs respectifs et l'autre capacité est reliée à une masse électrique du véhicule.

**9.** Dispositif selon l'une des revendications 1 à 8, **caractérisé en ce qu'**il est agencé pour répéter la mesure de la deuxième valeur complexe entre ladite électrode et le point de référence de circuit électrique de façon cyclique et, entre deux mesures successives de ladite deuxième valeur complexe, exécuter une mesure de calibration.

**10.** Dispositif selon la revendication 1 ou selon l'une des revendications 2 ou 4-9 lorsque celle-ci dépend de la revendication 1, **caractérisé en ce que** le dispositif de mesure (24) est agencé pour calculer une valeur de capacité entre ladite électrode (20) et le point de référence de circuit électrique, à partir de ladite deuxième valeur complexe mesurée puis corrigée, par application d'un gain de mise à l'échelle G à la partie imaginaire de ladite deuxième valeur mesurée puis corrigée, le gain G étant donné par la relation

$$G = \frac{|R_{C1} - R_{C2}|}{R_{C1} \times R_{C2} \times \|\overline{C_{R\_C1}C_{R\_C2}}\|}$$

où

- $R_{C1}$ représente une première résistance de calibration ;
- $R_{C2}$ représente une deuxième résistance de calibration ;
- $C_{R\_C1}$ représente un point, dans un plan complexe d'admittance, correspondant à la mesure d'une valeur complexe de la première résistance de calibration $R_{C1}$ ;
- $C_{R\_C2}$ représente un point, dans ledit plan complexe d'admittance, correspondant à la mesure d'une valeur complexe de la deuxième résistance de calibration $R_{C2}$.

**11.** Dispositif selon l'une des revendications précédentes, **caractérisé en ce que** le dispositif de mesure (24) comprend un circuit de mesure de courant.

**12.** Volant de véhicule automobile équipé du dispositif capacitif de détection (100) selon l'une des revendications précédentes.

**13.** Siège de véhicule automobile équipé du dispositif capacitif de détection (100) selon l'une des revendications 1 à 11.

**14.** Véhicule automobile comprenant un volant selon la revendication 12 et/ou un siège selon la revendication 13.

**Patentansprüche**

**1.** Kapazitive Erfassungsvorrichtung (100) zum Erfassen der Anwesenheit einer Person in der Nähe oder in Kontakt mit einer Komponente (1) eines Kraftfahrzeugs, die mit einem kapazitiven Sensor ausgestattet ist, die Vorrichtung umfassend:

- mindestens eine Elektrode (20) des kapazitiven Sensors,
- eine Spannungsquelle (23), die zum Abgeben einer Wechselspannung angeordnet ist,
- eine Vorrichtung (24) zum Messen eines komplexen Werts, der einer Impedanz oder einer Admittanz entspricht, zwischen der Elektrode (20) und einem Referenzpunkt einer elektrischen Schaltung;
- einen ersten Kalibrierungswiderstand ($R_{c1}$, $R_{c2}$) zum Kalibrieren der Messung eines komplexen Werts der Impedanz oder der Admittanz;

wobei:

die kapazitive Erfassungsvorrichtung eine Schaltvorrichtung (26, SW1-SW6) umfasst, die angeordnet ist zum:

- Verbinden der Spannungsquelle (23) mit der Elektrode (20), in einem Messmodus,
- Verbinden der Spannungsquelle (23) mit dem ersten Kalibrierungswiderstand ($R_{c1}$, $R_{c2}$) und Trennen der Elektrode (20) von der Spannungsquelle, in einem Kalibrierungsmodus, und die Messvorrichtung (24) angeordnet ist zum:
- Messen eines ersten komplexen Werts des ersten Kalibrierungswiderstands ($R_{C1}$, $R_{C2}$) während eines Betriebs in dem Kalibrierungsmodus;
- Messen eines zweiten komplexen Werts zwischen der Elektrode (20) und dem Referenzpunkt der elektrischen Schaltung während eines Betriebs in dem Messmodus; und
- Korrigieren des gemessenen zweiten komplexen Werts in Abhängigkeit des gemessenen ersten komplexen Werts,

**dadurch gekennzeichnet, dass** die kapazitive Erfassungsvorrichtung (100) ferner einen zweiten Kalibrierungswiderstand ($R_{C1}$, $R_{C2}$) umfasst,

**dass** der erste und der zweite Kalibrierungswiderstand ($R_{C1}$, $R_{C2}$) unterschiedliche Widerstandswerte aufweisen,

und **dadurch, dass** die zwei Kalibrierungswiderstände ($R_{C1}$, $R_{C2}$) parallel geschaltet sind und die Schaltvorrichtung (26, SW1-SW6) zum wahlweisen Verbinden der Spannungsquelle (23) mit dem einen oder dem anderen der zwei Widerstände ($R_{C1}$, $R_{C2}$) angeordnet ist.

2. Vorrichtung nach dem vorstehenden Anspruch,
**dadurch gekennzeichnet, dass** der erste Kalibrierungswiderstand ($R_{C1}$) einen Widerstandswert zwischen 1 kΩ und 20 kΩ besitzt und der zweite Kalibrierungswiderstand ($R_{C2}$) einen Widerstandswert zwischen 10 kΩ und 200 kΩ besitzt.

3. Kapazitive Erfassungsvorrichtung (100) zum Erfassen der Anwesenheit einer Person in der Nähe oder in Kontakt mit einer Komponente (1) eines Kraftfahrzeugs, die mit einem kapazitiven Sensor ausgestattet ist, die Vorrichtung umfassend:

- mindestens eine Elektrode (20) des kapazitiven Sensors,
- eine Spannungsquelle (23), die zum Abgeben einer Wechselspannung angeordnet ist,
- eine Vorrichtung (24) zum Messen eines komplexen Werts, der einer Impedanz oder einer Admittanz entspricht, zwischen der Elektrode (20) und einem Referenzpunkt der elektrischen Schaltung;
- einen ersten Kalibrierungswiderstand ($R_{c1}$, $R_{c2}$) zum Kalibrieren der Messung eines komplexen Werts der Impedanz oder der Admittanz;

wobei:

die kapazitive Erfassungsvorrichtung eine Schaltvorrichtung (26, SW1-SW6) umfasst, die angeordnet ist zum:

- Verbinden der Spannungsquelle (23) mit der Elektrode (20), in dem Messmodus,
- Verbinden der Spannungsquelle (23) mit dem ersten Kalibrierungswiderstand ($R_{c1}$, $R_{c2}$) und Trennen der Elektrode (20) von der Spannungsquelle, in dem Kalibrierungsmodus,

und die Messvorrichtung (24) angeordnet ist zum:

- Messen eines ersten komplexen Werts des ersten Kalibrierungswiderstands ($R_{C1}$, $R_{C2}$) während eines Betriebs in dem Kalibrierungsmodus;
- Messen eines zweiten komplexen Werts zwischen der Elektrode (20) und dem Referenzpunkt der elektrischen Schaltung während eines Betriebs in dem Messmodus; und
- Korrigieren des gemessenen zweiten komplexen Werts in Abhängigkeit des gemessenen ersten komplexen Werts,

**dadurch gekennzeichnet, dass** die kapazitive Erfassungsvorrichtung (100) ferner einen zweiten Kalibrierungswiderstand ($R_{C1}$, $R_{C2}$) umfasst,

**dass** der erste und der zweite Kalibrierungswiderstand ($R_{C1}$, $R_{C2}$) identische Werte aufweisen, und **dass** die zwei Kalibrierungswiderstände (Rci, $R_{C2}$) in Reihe geschaltet sind und die Schaltvorrichtung (26, SW1-SW6) zum wahlweisen Verbinden der Spannungsquelle (23) mit einem ersten Widerstandsdipol, umfassend nur einen der zwei Kalibrierungswiderstände (Rci, $R_{C2}$), oder mit einem zweiten Widerstandsdipol angeordnet ist, umfassend die zwei Kalibrierungswiderstände ($R_{C1}$, $R_{C2}$).

4. Vorrichtung nach einem der vorstehenden Ansprüche,
**dadurch gekennzeichnet, dass** die Messvorrichtung (24) zum Anwenden einer Versatzkorrektur auf den zweiten gemessenen komplexen Wert angeordnet ist, wobei die Versatzkorrektur einer Translation gemäß einem Vektor $\overrightarrow{S_{OC}O}$ in einer komplexen Ebene entspricht, wobei der Punkt Soc ein Punkt der komplexen Ebene ist, der einem komplexen Wert einer Leerlaufschaltungsadmittanz oder -impedanz der kapazitiven Erfassungsvorrichtung entspricht, und der Punkt O dem Ursprung der komplexen Ebene entspricht.

5. Vorrichtung nach dem vorstehenden Anspruch,
**dadurch gekennzeichnet, dass** die Messvorrichtung (24) zum Berechnen des komplexen Werts der Leerlaufschaltungsimpedanz oder -admittanz (SOC) der kapazitiven Erfassungsvorrichtung aus zwei komplexen Werten angeordnet ist, die mithilfe der zwei Kalibrierungswiderstände (Rci, $R_{C2}$) gemessen werden.

6. Vorrichtung nach dem vorstehenden Anspruch,
**dadurch gekennzeichnet, dass** die Messvorrichtung (24) zum Messen eines komplexen Werts der Leerlaufschaltungsimpedanz oder -admittanz (COC) und Vergleichen des gemessenen komplexen Werts der Leerlaufschaltungsimpedanz oder -admittanz und des berechneten komplexen Werts der Leerlaufschaltungsimpedanz oder -admittanz (SOC) angeordnet ist, um den ordnungsgemäßen Betrieb der kapazitiven Erfassungsvorrichtung zu überprüfen.

7. Vorrichtung nach einem der Ansprüche 1 bis 6,
**dadurch gekennzeichnet, dass** die Messvorrichtung (24) zum Anwenden einer Phasenkorrektur (E13) auf den zweiten gemessenen komplexen Wert angeordnet ist, wobei die Phasenkorrektur einer Drehung um einen Winkel $\alpha$ zwischen einer Geraden, die durch zwei Punkte in einer komplexen Ebene definiert ist, die aus zwei komplexen Werten der Impedanz oder der Admittanz erhalten werden, die mithilfe der zwei Kalibrierungswiderstände gemessen werden, und einer Achse der Widerstände oder Leitwerte der komplexen Ebene entspricht.

8. Vorrichtung nach einem der vorstehenden Ansprüche,
**dadurch gekennzeichnet, dass** sie ein Paar Schutzkapazitäten (CP1, CP2) in Reihe umfasst, wobei das Paar von Kapazitäten parallel zu Kalibrierungswiderständen (Rci, $R_{C2}$) geschaltet ist, und dass eine der zwei Kapazitäten an ihren Anschlüssen über zwei jeweilige Schalter mit der Spannungsquelle (23) verbunden ist und die andere Kapazität mit einer elektrischen Masse des Fahrzeugs verbunden ist.

9. Vorrichtung nach einem der Ansprüche 1 bis 8,
**dadurch gekennzeichnet, dass** sie zum zyklischen Wiederholen der Messung des zweiten komplexen Wertes zwischen der Elektrode und dem Referenzpunkt der elektrischen Schaltung und, zwischen zwei aufeinanderfolgenden Messungen des zweiten komplexen Wertes, Ausführen einer Kalibrierungsmessung angeordnet ist.

10. Vorrichtung nach Anspruch 1 oder nach einem der Ansprüche 2 oder 4 bis 9, wenn dieser von Anspruch 1 abhängt,
**dadurch gekennzeichnet, dass** die Messvorrichtung (24) zum Berechnen eines Kapazitätswerts zwischen der Elektrode (20) und dem Referenzpunkt der elektrischen Schaltung aus dem zweiten gemessenen und dann korrigierten komplexen Wert durch Anwenden einer Skalierungsverstärkung G auf den imaginären Teil des zweiten gemessenen und dann korrigierten Wertes angeordnet ist, wobei die Verstärkung G durch die Beziehung gegeben ist:

$$G = \frac{|R_{C1} - R_{C2}|}{R_{C1} \times R_{C2} \times \left\| \overline{C_{R\_C1}C_{R\_C2}} \right\|}$$

worin

- $R_{C1}$ einen ersten Kalibrierungswiderstand darstellt;
- $R_{C2}$ einen zweiten Kalibrierungswiderstand darstellt;

- $C_{R\_C1}$ einen Punkt in einer komplexen Admittanzebene darstellt, der der Messung eines komplexen Werts des ersten Kalibrierungswiderstands $R_{C1}$ entspricht;
- $C_{R\_C2}$ einen Punkt in der komplexen Admittanzebene darstellt, der der Messung eines komplexen Werts des zweiten Kalibrierungswiderstands $R_{C2}$ entspricht.

11. Vorrichtung nach einem der vorstehenden Ansprüche,
    **dadurch gekennzeichnet, dass** die Messvorrichtung (24) eine Strommessschaltung umfasst.

12. Kraftfahrzeuglenkrad, das mit der kapazitiven Erfassungsvorrichtung (100) nach einem der vorstehenden Ansprüche ausgestattet ist.

13. Kraftfahrzeugsitz, der mit der kapazitiven Erfassungsvorrichtung (100) nach einem der Ansprüche 1 bis 11 ausgestattet ist.

14. Kraftfahrzeug, umfassend ein Lenkrad nach Anspruch 12 und/oder einen Sitz nach Anspruch 13.

**Claims**

1. A capacitive detection device (100) for detecting the presence of a person near or in contact with a component (1) of a motor vehicle, which component is provided with a capacitive sensor, said device comprising

   - at least one electrode (20) of the capacitive sensor,
   - a voltage source (23) arranged to deliver an alternating voltage,
   - a device (24) for measuring a complex value, corresponding to an impedance or an admittance, between said electrode (20) and an electrical circuit reference point;
   - a first calibration resistor ($R_{c1}$, $R_{c2}$) for calibrating the measurement of a complex impedance or admittance value;

   wherein:

   said capacitive detection device comprises a switching device (26, SW1-SW6) arranged to

   - connect the voltage source (23) to said electrode (20), in a measurement mode,
   - connect the voltage source (23) to the first calibration resistor ($R_{c1}$, $R_{c2}$) and disconnect said electrode (20) from the voltage source, in a calibration mode, and the measuring device (24) is arranged to
   - measure a first complex value of the first calibration resistor ($R_{c1}$, $R_{c2}$), during operation in the calibration mode;
   - measure a second complex value between said electrode (20) and said electrical circuit reference point, during operation in the measurement mode, and
   - correct the second measured complex value on the basis of the first measured complex value,

   **characterized in that** the capacitive detection device (100) further comprises a second calibration resistor ($R_{C1}$, $R_{C2}$),
   **in that** the first and the second calibration resistor ($R_{C1}$, $R_{C2}$) have different resistive values,
   and **in that** the two calibration resistors ($R_{C1}$, $R_{C2}$) are mounted in parallel and the switching device (26, SW1-SW6) is arranged to selectively connect the voltage source (23) to one or the other of the two resistors ($R_{C1}$, $R_{C2}$).

2. The device according to the preceding claim, **characterized in that** the first calibration resistor ($R_{C1}$) has a resistive value of between 1 kΩ and 20 kΩ and the second calibration resistor ($R_{C2}$) has a resistive value of between 10 kΩ and 200 kΩ.

3. A capacitive detection device (100) for detecting the presence of a person near or in contact with a component (1) of a motor vehicle, which component is provided with a capacitive sensor, said device comprising

   - at least one electrode (20) of the capacitive sensor,
   - a voltage source (23) arranged to deliver an alternating voltage,
   - a device (24) for measuring a complex value, corresponding to an impedance or an admittance, between said electrode (20) and an electrical circuit reference point;
- a first calibration resistor ($R_{c1}$, $R_{c2}$) for calibrating the measurement of a complex impedance or admittance value;

wherein:

said capacitive detection device comprises a switching device (26, SW1 - SW6) arranged to

- connect the voltage source (23) to said electrode (20), in a measurement mode,
- connect the voltage source (23) to the first calibration resistor ($R_{c1}$, $R_{c2}$) and disconnect said electrode (20) from the voltage source, in a calibration mode,

and the measuring device (24) is arranged to

- measure a first complex value of the first calibration resistor ($R_{C1}$, $R_{C2}$), during operation in the calibration mode;
- measure a second complex value between said electrode (20) and said electrical circuit reference point, during operation in the measurement mode, and
- correct the second measured complex value on the basis of the first measured complex value,

**characterized in that** the capacitive detection device (100) further comprises a second calibration resistor ($R_{C1}$, $R_{C2}$),
**in that** the first and the second calibration resistor ($R_{C1}$, $R_{C2}$) have identical values,
and **in that** the two calibration resistors (Rci, $R_{C2}$) are mounted in series and the switching device (26, SW1-SW6) is arranged to selectively connect the voltage source (23) to a first resistive dipole comprising only one of the two calibration resistors ($R_{C1}$, $R_{C2}$) or to a second resistive dipole comprising the two calibration resistors ($R_{C1}$, $R_{C2}$).

4. The device according to any of the preceding claims, **characterized in that** the measuring device (24) is arranged to apply an offset correction to the second measured complex value, said offset correction corresponding to a translation according to a vector $Soc$O in a complex plane, the point Soc being a point of the complex plane corresponding to an open-circuit complex admittance or impedance value of the capacitive detection device and the point O corresponding to the origin of the complex plane.

5. The device according to the preceding claim, **characterized in that** the measuring device (24) is arranged to calculate said open-circuit complex impedance or admittance value (SOC) of the capacitive detection device from two complex values measured using the two calibration resistors ($R_{C1}$, $R_{C2}$).

6. The device according to the preceding claim, **characterized in that** the measuring device (24) is arranged to measure an open-circuit complex impedance or admittance value (COC) and to compare said measured open-circuit complex impedance or admittance value and said calculated open-circuit complex impedance or admittance value (SOC) in order to verify the correct operation of the capacitive detection device.

7. The device according to any of claims 1 to 6, **characterized in that** the measuring device (24) is arranged to apply a phase correction (E13) to the second measured complex value, said phase correction corresponding to a rotation according to an angle $\alpha$ between a straight line defined by two points in a complex plane, which points are obtained from two complex impedance or admittance values measured using the two calibration resistors, and an axis of the resistances or conductances of the complex plane.

8. The device according to any of the preceding claims, **characterized in that** it comprises a pair of protection capacitors (CP1, CP2) in series, said pair of capacitors being mounted in parallel with the calibration resistors ($R_{C1}$, $R_{C2}$), and **in that** one of the two capacitors is connected at its terminals to the voltage source (23) by means of two respective switches and the other capacitor is connected to an electrical ground of the vehicle.

9. The device according to any of claims 1 to 8, **characterized in that** it is arranged to repeat the measurement of the second complex value between said electrode and the electrical circuit reference point in a cyclic manner and, between two successive measurements of said second complex value, perform a calibration measurement.

10. The device according to claim 1 or according to any of claims 2 or 4-9 when dependent on claim 1, **characterized in that** the measuring device (24) is arranged to calculate a capacitance value between said electrode (20) and the electrical circuit reference point, from said second measured and then corrected complex value, by applying a scaling gain G to the imaginary part of said second measured and then corrected value, the gain G being given by the equation

$$G = \frac{|R_{C1} - R_{C2}|}{R_{C1} \times R_{C2} \times \left\| C_{R\_C1} C_{R\_C2} \right\|}$$

where

- $R_{C1}$ represents a first calibration resistor;
- $R_{C2}$ represents a second calibration resistor;
- $C_{R\_C1}$ represents a point, in a complex admittance plane, corresponding to the measurement of a complex value of the first calibration resistor $R_{C1}$;
- $C_{R\_C2}$ represents a point, in said complex admittance plane, corresponding to the measurement of a complex value of the second calibration resistor $R_{C2}$.

11. The device according to any of the preceding claims, **characterized in that** the measuring device (24) comprises a current measuring circuit.

12. A motor vehicle steering wheel provided with the capacitive detection device (100) according to any of the preceding claims.

13. A motor vehicle seat provided with the capacitive detection device (100) according to any of claims 1 to 11.

14. A motor vehicle comprising a steering wheel according to claim 12 and/or a seat according to claim 13.

**FIG. 1**

**FIG. 3**

**FIG. 2**

**FIG. 4**

**FIG. 5**

E00 → SW1, SW2 ouverts
SW5, SW6 fermés

E01 → MES $CR_{10}$

E02 → MES $CR_{100}$

E07 → MES $C_{OC}$

E03 → $S_{OC}$

E04 → $\overrightarrow{S_{OC}O}$

E0

E05 → $C_{R10\_S}$

E06 → $G$

E08 → DIAG $[S_{OC}, C_{OC}]$

**FIG. 6.1**

E10 — SW1, SW2 fermés
SW5, SW6 ouverts

E11 — MES Zx (Rx,Cx) ➜ M

E12 — M ➜ $M_S$

E13 — $M_S$ ➜ $M_{S,R}$

E14 — $C = \dfrac{G.y}{2.\pi.f}$

E15 — DETECT

E1

**FIG. 6.2**

CAL$_{R10K}$
CAL$_{R100K}$
MES Zx

**FIG. 7**

**EP 3 814 196 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20150367751 A
- US 2017355337 A
- WO 2016055667 A1
- EP 0802420 A2